# EUROPÄISCHE PATENTSCHRIFT

(11) **EP 3 774 284 B1**
(45) Veröffentlichungstag und Bekanntmachung des Hinweises auf die Patenterteilung: **20.04.2022**
(21) Anmeldenummer: 19715803.3
(22) Anmeldetag: 27.03.2019
(51) Int. Cl.: B33Y 30/00, B33Y 70/00, B29C 64/124, B29C 64/112, B29C 64/194, A61K 9/20, B33Y 10/00, B33Y 80/00, A61L 27/16, A61L 27/54

(54) **VORRICHTUNG ZUR HERSTELLUNG VON 3D-GEDRUCKTEN WIRKSTOFFFREISETZUNGSSYSTEMEN MIT WIRKSTOFFDEPOTS, SOWIE VERFAHREN ZUR HERSTELLUNG VON 3D-GEDRUCKTEN WIRKSTOFFFREISETZUNGSSYSTEMEN**
DEVICE FOR PRODUCING 3D-PRINTED ACTIVE SUBSTANCE-RELEASING SYSTEMS WITH ACTIVE SUBSTANCE DEPOTS, AND METHOD FOR PRODUCING 3D-PRINTED ACTIVE SUBSTANCE-RELEASING SYSTEMS
DISPOSITIF SERVANT À FABRIQUER DES SYSTÈMES DE LIBÉRATION DE PRINCIPES ACTIFS À IMPRESSION 3D COMPRENANT DES DÉPÔTS DE PRINCIPES ACTIFS, AINSI QUE PROCÉDÉ SERVANT À FABRIQUER DES SYSTÈMES DE LIBÉRATION DE PRINCIPES ACTIFS À IMPRESSION 3D

(30) Priorität: 29.03.2018 DE 102018107585
(43) Veröffentlichungstag der Anmeldung: 17.02.2021
(73) Patentinhaber: Universität Rostock, 18055 Rostock (DE)
(72) Erfinder: SEITZ, Hermann, 18055 Rostock (DE); MARTZMOHR, Roland, 18246 Neuendorf (DE); GRABOW, Niels, 18055 Rostock (DE); TESKE, Michael, 18119 Rostock (DE); EICKNER, Thomas, 18059 Rostock (DE); MAU, Robert, 18059 Rostock (DE); RIESS, Alexander, 18146 Rostock (DE); SCHMITZ, Klaus-Peter, 18119 Rostock (DE)
(74) Vertreter: Grünbaum, Annekathrin
(86) Internationale Anmeldenummer: PCT/EP2019/057734
(87) Internationale Veröffentlichungsnummer: WO 2019/185725

(56) Entgegenhaltungen:
- EP-A1- 2 532 349
- WO-A1-2009/139395
- WO-A1-2017/040156

## Beschreibung

Die vorliegende Erfindung bezieht sich auf eine Vorrichtung gemäß Anspruch 1 zur Herstellung von 3D-gedruckten Wirkstofffreisetzungssystemen sowie auf ein Verfahren gemäß Anspruch 17 zur Herstellung von 3D-gedruckten Wirkstofffreisetzungssystemen.

Die Erfindung ist den Gebieten der additiven Fertigungsverfahren bzw. Formgebungsverfahren für wirkstofffreisetzende Medizinprodukte, Implantattechnologie, Biomaterialien und Kombinationsprodukte zuzuordnen.

Im Stand der Technik sind Wirkstofffreisetzungssysteme (engl.: Drug-Delivery-Systeme) bekannt. Bei diesen erfolgt die Wirkstofffreisetzung über die Außen äche eines Grundkörpers bzw. über eine ächige Beschichtung - der Wirkstoff wird dabei durch einen Träger, einen sogenannten Carrier, getragen. Der Carrier ist meist ein Polymer. Es gibt jedoch auch Verfahren, bei welchen der Carrier ein Implantat ist, auf dessen Oberfläche ein Wirkstoff aufgebracht wird. Der maximalen Beladung und Konzentration des Wirkstoffs sind dabei Grenzen bei der Löslichkeit im polymeren Wirkstoffträger und dessen Schichtintegrität gesetzt. Die Freisetzung erfolgt dabei häu g durch Diffusion und wird durch die Eigenschaften des wirkstofftragenden Polymers und des Wirkstoffs selbst definiert. In der Folge können lokal nur für kurze Zeiträume medizinisch relevante Dosen freigesetzt werden.

In den Druckschriften WO002013182913A1 und EP1103368A1 sind Stereolithographische Verfahren für den Druck von pharmazeutischen Produkten bekannt. Diese weisen im Vergleich zu pulverbettbasierten oder extrusionsbasierten Verfahren eine höhere Präzision auf und ermöglichen ein gezieltes Einstellen der Abbaukinetiken. Die offenbarten stereolithografischen Verfahren arbeiten mit einer fokussierten Strahlenquelle, wie beispielsweise einem Laser. Bei dieser Technologie wird mit einem Photopolymerbad in einer Wanne gearbeitet, eine fokussierte Lichtquelle wie ein Laser härtet mit einer bestimmten Eindringtiefe das Photopolymer selektiv aus, daraufhin streicht ein Rakel eine neue Schicht Photopolymer auf, die erneut durch die fokussierte Lichtquelle bauteilspezi sch ausgehärtet wird. Dies wird Schicht für Schicht bis zum fertigen Bauteil wiederholt. Durch die Präzision der Lichtstrahlquelle lässt sich die Polymerisation des Werkstoffes ortsgenau einstellen. Zudem weist das Verfahren die notwendige Genauigkeit auf, kleinste Reservoirgeometrien zu fertigen. In der Druckschrift DE102013021961A1 ist zudem ein stereolithografisches Verfahren mit mehr als einer unabhängigen Strahlenquelle offenbart. Derartige Verfahren realisieren den Schichtauftrag des (initial flüssigen) Photopolymers aus einem Flüssigkeitsbad heraus, wobei die Fließeigenschaften des Photopolymers sowie Rakel bzw. Walzen genutzt werden.

Ebenfalls bekannt im Stand der Technik sind die ebenfalls auf die Photopolymerisation zurückgreifenden 3D-Druck-Verfahren Polyjetting (PJ) bzw. Multijetting (MJM) welche für den Materialauftrag tropfenerzeugende Druckköpfe verwenden, beispielsweise Inkjet-Druckköpfe. Mit Erst- und Hauptanwendungen im Farb- und Fotodruck, wird die Technologie auch für eine Vielzahl von pharmazeutischen und medizintechnischen Aufgaben eingesetzt. Die Anwendung der Inkjet-Technologie ist im 3D-Druck etabliert. Dies betrifft insbesondere pulverbasierten 3D-Druck, als auch die Verfahren PJ bzw. MJM. Bei den letztgenannten Verfahren werden Photopolymere durch Inkjet-Druckköpfe ortsselektiv in mehreren Schichten aufgetragen und durch Photopolymerisation Schicht für Schicht ausgehärtet. Die Verarbeitung mehrerer Materialien simultan ist möglich, üblich ist die simultane Verarbeitung von zumindest zwei Materialien: Baumaterial und Supportmaterial. Die Belichtung zur Photopolymerisation erfolgt hierbei durch eine geeignete Strahlungsquellen. Die Druckschriften JP002016196104A, JP002016196104A und CN000205818475U offenbaren verfahrbare Inkjet-Druckköpfe und eine Strahlungsquelle für eine Photopolymerisation. Die Druckköpfe erfüllen hierbei jedoch ausschließlich die Funktion des Auftragens des Baumaterials, welches ein reines Photopolymer ist. Eine simultane Verarbeitung eines funktionellen Wirkstoffes ist nicht offenbart.

In der Druckschrift JP002015143032A ist jedoch ein Verfahren offenbart, welches die Inkjet-Druckköpfe nicht ausschließlich für die Verarbeitung von Photopolymeren einsetzt. In diesem Verfahren erfolgt nach Aushärtung von Photopolymeren durch Laserlichtquellen ein Auftrag von Farbstoff durch einen Inkjet-Druckköpfe auf die ausgehärteten Materialschichten. Hierbei wird jedoch der Farbstoff auf ausgehärtete Schichten aufgetragen. Dies geschieht auf die gesamte ausgehärtete Schicht und nicht in gezielte Reservoirstrukturen. Zudem ist nicht beschrieben, dass der Farbstoff durch eine gezielte Photopolymerisation funktionell mit dem Grundkörper verbunden wird. Eine gezielte Freisetzung des Farbstoffes ist ebenfalls nicht beschrieben.

Die Anwendung von Wirkstoffreservoirs auf Implantatoberflächen samt Beladungsprozess durch Tropfenerzeugung mittels Inkjet-Verfahren ist in der Druckschrift DE102012208615A1 offenbart. Die Herstellung der Reservoirs und die Wirkstoffbeladung sind getrennte Prozesse, die nacheinander ablaufen müssen. Beispielsweise erfolgt im ersten Schritt eine Laserbohrung der Reservoirs. Nach Reinigung und Nachbehandlung folgt im zweiten Schritt die Beladung mit Wirkstoff durch Tropfenerzeuger. Ein Fertigungsprozess zur Herstellung von hochpräzisen formkomplexen Bauteilen mit ortsselektiven wirkstoffbeladenen Reservoirs, die direkt während eines zusammenhängenden Herstellungsprozesses in das Bauteil eingebaut werden und zudem eine gezielte Vernetzung der Wirkstoffe mit dem Basispolymer des Bauteils aufweisen, ist nicht beschrieben.

In der Offenbarungsschrift WO 2017/040156 A1 ist ein hybrides 3D-Drucksystem offenbart, worin eine extrusionsbasierte 3D-Drucktechnik zum Aufbau eines Gerüstes/Polymergitters verwendet wird, welches anschließend in eine (photopolymerisierbare) Prepolymerlösung eingetaucht wird. Die Prepolymerlösung kann ein Wirkstoffgemisch enthalten und bildet so ein Wirkstofffreisetzungssystem.

Die Offenbarungsschrift EP 2 532 349 A1 offenbart einen 3D-Druck von Wirkstofffreisetzungssystem nach dem Inkjet-Prinzip, durch gezielte Abscheidung verschiedener Materialien (Wirkstoffe und Hilfsstoffe) aus Inkjet-Köpfen.

Die WO 2009/139395 A1 offenbart eine 3D-Drucktechnik, bei welcher Flüssigharzschichten in einer Wanne gebildet werden und jede Schicht ortsselektiv durch Aufbringen eines flüssigen Reaktanten mittels Inkjet polymerisiert wird.

Der Stand der Technik bietet kein hochauflösendes 3D-Druckverfahren, mit dem sich Wirkstofffreisetzungssysteme mit ortsselektiven Wirkstoffdepots mit wirkstoffschonender, gesteuerter Ankopplung in einem zusammenhängenden Prozess herstellen lassen.

### Darstellung der Erfindung

Der Erfindung liegt die Aufgabe zugrunde, eine Vorrichtung bereitzustellen, welche die Nachteile des Standes der Technik beseitigt und den Bau von hochauflösenden formbeliebigen Wirkstofffreisetzungssystemen mit ortsselektiven Wirkstoffdepots zulässt. Der Erfindung liegt auch die Aufgabe zu Grunde ein Verfahren bereitzustellen, welches die Nachteile des Standes der Technik beseitigt und den Bau von hochauflösenden formbeliebigen Wirkstofffreisetzungssystemen mit ortsselektiven Wirkstoffdepots zulässt.

Die Lösung der Aufgaben erfolgt mit den Merkmalen des Anspruchs 1 und des Anspruchs 16, wobei die Unteransprüche weitere Ausgestaltungen beschreiben.

Die Vorrichtung zur Herstellung eines 3D-gedruckten Wirkstofffreisetzungssystems mit Wirkstoffdepots gemäß Anspruch 1 weist mindestens einen Inkjet-Druckkopf und mindestens eine Strahlungsquelle auf. Eine Wanne zur Aufnahme des zu verarbeitenden Basismaterials weist die Vorrichtung ebenfalls auf. Das Basismaterial kann ein Monomer, aber auch ein Polymer oder ein Mischung von Monomer und Polymer sein. Die Verwendung mehrerer verschiedener Monomere oder Polymere ist auch denkbar. Beispiele hierfür sind kommerziell erhältliche Polyethylenglycole, Poly(ethylenglycol)diacrylate und Gelatinemethacrylate.

Weiterhin weist die Vorrichtung eine Bauplattform auf. Die Bauplattform ist innerhalb der Wanne derart angeordnet, dass diese in vertikaler Richtung beweglich gelagert ist.

Die mindestens eine Strahlungsquelle ist derart ausgebildet, dass diese im Betrieb eine Polymerisation einer Schicht des Basismaterials auf der Bauplattform bewirkt.

Erfindungsgemäß ist der mindestens eine Inkjet-Druckkopf derart ausgebildet, dass dieser im Betrieb ein Wirkstoff-Gemisch, welches mindestens einen Wirkstoff aufweist, in das Basismaterial ortsselektiv inkorporiert.

Weitere Inkjet-Druckköpfe, welche die Vorrichtung aufweist sind denkbar, wobei jeder Inkjet-Druckkopf ein Wirkstoff-Gemisch in das Basismaterial ortsselektiv inkorporieren kann. Das Wirkstoff-Gemisch, welches ein Inkjet-Druckkopf ortsselektiv in das Basismaterial inkorporiert, kann verschieden sein zu dem Wirkstoff-Gemisch eines anderen Inkjet-Druckkopfes.

Bevorzugt ist der mindestens eine Inkjet-Druckkopf in einer Ebene oberhalb der Wanne mit dem Basismaterial in x-y-Richtung verfahrbar angeordnet. Alternativ ist eine Verfahrbarkeit der Bauplattform denkbar. Mittels der Verfahrbarkeit der mindestens einen Inkjet-Druckkopf oder der Verfahrbarkeit der Bauplattform wird erreicht, dass das Wirkstoff-Gemisch ortsselektiv im Basismaterial inkorporiert wird. Zusätzlich ist eine Verfahrbarkeit des Inkjet-Druckkopfs in einer weiteren Längsrichtung (z-Richtung) denkbar.

Das Wirkstoff-Gemisch weist bevorzugt mindestens ein Monomer und/oder mindestens ein Polymer auf. Die Verwendung mehrerer verschiedener Monomere und/oder Polymere ist auch denkbar. Weiterhin weist das Wirkstoff-Gemisch mindestens einen Photoinitiator und/oder mindestens einen Vernetzer, sogenannte Crosslinker, auf. Die Verwendung mehrerer Photoinitiatoren und/oder mehrerer Vernetzer ist jedoch auch denkbar. Photoinitiatoren sind chemische Verbindungen, die nach Absorption von Licht in reaktive Moleküle zerfallen, die ihrerseits die Polymerisationsreaktion auslösen. Vernetzer sind Monomere oder Polymere mit mindestens drei Funktionalitäten (z.B. mehr als zwei funktionellen Gruppen oder mehr als eine Doppelbindung), welche durch Polymerisationsreaktionen reagieren können. Diese bewirken eine Kettenverzweigung und damit eine Vernetzung der kettenförmigen Polymermoleküle.

Auch das Basismaterial weist bevorzugt mindestens einen Photoinitiator und/oder mindestens einen Vernetzer auf. Das Basismaterial mit einem Vernetzer auszustatten bewirkt, dass das Wirkstoff-Gemisch nicht extra Vernetzer aufweisen muss. Dadurch bewirkt auch die Polymerisation des Basismaterials, dass auch der Wirkstoff mit dem Basismaterial vernetzt wird.

Das Monomer des Basismaterials und/oder des Wirkstoff-Gemisches kann ein Photomonomer sein. Das Polymer des Basismaterials und/oder des Wirkstoff-Gemisches kann ein Photopolymer sein. Photomonomere bzw. Photopolymere polymerisieren, wenn diese mit einer gewissen Strahlungsintensität bestrahlt werden.

Das Monomer des Basismaterials und/oder des Wirkstoff-Gemisches und/oder das Polymer des Basismaterials und/oder des Wirkstoff-Gemisches kann/können ein Vernetzer/Vernetzer sein.

Das Monomer des Basismaterials und/oder des Wirkstoff-Gemisches und/oder das Polymer des Basismaterials und/oder des Wirkstoff-Gemisches kann/können eine definierte Menge an Photoinitiatoren aufweisen.

Die mindestens eine Strahlungsquelle ist derart ausgebildet, dass diese eine Polymerisation des Wirkstoff-Gemisches bewirkt und/oder eine Vernetzung des Wirkstoff-Gemisches mit dem Basismaterial bewirkt.

Anstatt der mindestens einen Strahlungsquelle, welche für eine Polymerisation des Basismaterials als auch des Wirkstoff-Gemisches verwendet wird, kann die Vorrichtung auch eine zweite Strahlungsquelle oder mehrere Strahlungsquellen aufweisen. Während die mindestens eine Strahlungsquelle das Basismaterial polymerisiert, ist die zweite Strahlungsquelle dann derart ausgebildet, dass diese eine Polymerisation des Wirkstoff- Gemisches bewirkt und/oder eine Vernetzung des Wirkstoff-Gemisches mit dem Baumaterial bewirkt. Weitere Strahlungsquellen, welche die Vorrichtung aufweist, für weitere Wirkstoff-Gemische sind denkbar und vorteilhaft, wenn unterschiedliche Wirkstoff-Gemische verschiedene Mono-/Polymere, eine unterschiedliche Anzahl an Photoinitiatoren und/oder verschiedene Vernetzer aufweisen. Jedes Wirkstoff-Gemisch kann dann durch unterschiedliche Strahlungsquellen polymerisiert und/oder vernetzt werden.

Bevorzugt ist der Inkjet-Druckkopf beheizbar. Hierfür kann der Inkjet-Druckkopf eine Heizvorrichtung aufweisen. Bevorzugt weist der Inkjet-Druckkopf ein Reservoir auf, in welchem das Wirkstoff-Gemisch vorrätig aufbewahrt ist. Alternativ kann auch das Reservoir beheizbar sein, indem auch das Reservoir eine Heizvorrichtung aufweist. Dadurch wird eine ideale Temperatur bereitgestellt, welche ermöglicht, dass eine Verarbeitung von Flüssigkeiten, welche bei Raumtemperatur zu viskos für einen Inkjet-Prozess sind, erfolgt. Das Beheizen mittels der Heizvorrichtung erfolgt dabei unter Beachtung der thermischen Stabilität des Wirkstoffes, welcher durch das Inkjet-Modul in das Basismaterial eingebracht wird.

Um ein Nachfließen des Wirkstoff-Gemisches zu ermöglichen ist das Reservoir selbstentleerend im Betrieb an dem Inkjet-Druckkopf angebracht. Die Selbstentleerung wird mittels Naturkräften, ohne weitere Kräfte, erreicht. Naturkräfte sind beispielsweise Gravitationskräfte oder Kapillarkräfte. Weitere Kräfte, beispielsweise hervorgerufen durch Pumpen, sind nicht erforderlich. Hierfür kann das Reservoir beispielsweise oberhalb des Inkjet-Modules angebracht sein, um durch einen so erzeugten Überdruck das Nachfließen des Wirkstoff-Gemisches zu erreichen. Eine Anordnung neben oder unterhalb des Inkjet-Druckkopfs ist jedoch auch denkbar.

Bevorzugt ist die Strahlungsquelle und/oder die zweite Strahlungsquelle und/oder jeder weitere Strahlungsquelle ein optoelektrisches Bauelement, wie beispielsweise eine Laserdiode, eine Lichtdiode, eine Leuchtdiode, oder ein optischer Resonator, wie beispielsweise ein Laser, oder eine Maskenbelichtungs-Vorrichtung, wie beispielsweise eine Bestrahlungsmaske.

Bevorzugt ist die Strahlungsquelle und/oder die zweite Strahlungsquelle derart ausgestaltet, dass diese in ihrer Strahlungsintensität einstellbar ist/sind. Bevorzugt weist die Vorrichtung hierfür eine Steuerung zur Ansteuerung der mindestens einen Strahlungsquelle und/oder der zweiten Strahlungsquelle und/oder jeder weiteren Strahlungsquelle auf, welche derart eingerichtet ist, dass verschiedene Strahlungscharakteristika bereitgestellt werden. Durch verschiedene Strahlungsintensitäten wird erreicht, dass verschiedene Wirkstoff-Gemische und/oder das Basismaterial, welche bei voneinander verschiedenen Strahlungsintensitäten vernetzen, bestrahlt werden können. Die Vernetzung basiert auf spaltbaren Bindungen, beispielsweise durch Hydrolyse spaltbare Bindungen, wodurch sich die Spaltung der Bindungen und somit die Wirkstofffreigabe steuern lässt.

Bevorzugt sind die Strahlungsquelle und jede weitere Strahlungsquelle derart ausgestaltet, dass diese voneinander verschiedene Wellenlängen für die Polymerisation des Basismaterials und/oder verschiedener Wirkstoff-Gemische erzeugen. Dass die Strahlungsquellen unterschiedliche Wellenlängen erzeugen, bewirkt, dass unterschiedliche Polymere oder Monomere für das Basismaterial als auch für das Wirkstoff-Gemisch oder die Wirkstoff-Gemische verwendet werden können. Dies wiederum bewirkt eine gesteuerte Polymerisation, was zu einer gezielten Freisetzung des Wirkstoffes führt.

Die Aufgabe wird auch gelöst durch ein Verfahren zur Herstellung eines 3D-gedruckten Wirkstofffreisetzungssystems mit Wirkstoffdepots gemäß Anspruch 16. Das Verfahren weist erfindungsgemäß folgende Verfahrensschritte auf, wobei die Verfahrensschritte nicht in der aufgezeigten Reihenfolge erfolgen müssen:

Bereitstellen einer Wanne, in welcher ein Basismaterial und eine Bauplattform angeordnet sind, wobei das Basismaterial ein Monomer und/oder Polymer ist. Beispiele hiefür sind kommerziell erhältliche Polyethylenglycole, Poly(ethylenglycol)diacrylate und Gelatinemethacrylate.

Polymerisieren ausgewählter Bereiche einer Schicht des Basismaterials auf der Bauplattform. Das Polymerisieren der ausgewählten Bereiche der Schicht des Basismaterials kann mittels einer Strahlungsquelle auf der Bauplattform erfolgen, wobei die Bauplattform innerhalb der Wanne derart angeordnet ist, dass die Bauplattform innerhalb der Wanne in vertikaler Richtung bewegbar gelagert ist. Eine andere bewegliche Lagerung der Bauplattform ist ebenfalls denkbar.

Absenken der Bauplattform, wobei umliegendes Basismaterial, welches nicht polymerisiert ist, nachfließt.

Glätten des nachgeflossenen Basismaterials, wobei das Basismaterial aktiv geglättet wird, beispielsweise mittels eines Rakels. Andere aktive Glättungsvorrichtungen sind jedoch auch denkbar. Eine Möglichkeit des Glättens bildet auch das passive Glätten, beispielsweise durch Abwarten, bis das nichtpolymerisierte Basismaterial komplett nachgeflossen ist und sich eine glatte Oberfläche gebildet hat. Ein aktives Glätten mittels einer Glättvorrichtung weist jedoch den Vorteil auf, dass eine Dicke der Schicht, welche polymerisiert wird, besser kontrollierbar ist.

Erfindungsgemäß wird mindestens ein Wirkstoff-Gemisch, welches mindestens einen Wirkstoff aufweist, mittels mindestens eines Inkjet-Druck-Verfahrens ortsselektiv in das Basismaterial und/oder das polymerisierte Basismaterial inkorporiert/eingebracht, um auf diese Weise Wirkstoffdepots im Wirkstofffreisetzungssystem zu erzeugen.

Durch das Absenken der Bauplattform können weitere Bereiche einer weiteren Schicht des Basismaterials polymerisiert werden. Dadurch wird schichtweise ein dreidimensionaler Körper aufgebaut.

Durch den schichtweisen Aufbau des dreidimensionalen Körpers und dem ortsselektiven Einbringen des Wirkstoff-Gemisches in die Schichten und/oder das Basismaterials wird ein Wirkstofffreisetzungssystem mit Wirkstoffdepots an bestimmten lokalen Stellen des Wirkstofffreisetzungssystems bereitgestellt.

Die Verwendung mehrerer Inkjet-Druckköpfe im Inkjet-Druckverfahren ist bevorzugt, um verschiedene Wirkstoff-Gemische, welche unterschiedliche Eigenschaften aufweisen, in das Basismaterial zu inkorporieren.

Bevorzugt ist mindestens ein Inkjet-Druckkopf in einer Ebene oberhalb der Wanne mit dem Basismaterial in x-y-Richtung verfahrbar angeordnet. Alternativ ist eine Verfahrbarkeit der Bauplattform denkbar. Mittels der Verfahrbarkeit des mindestens einen Inkjet-Druckkopfs oder die Verfahrbarkeit der Bauplattform wird erreicht, dass das Wirkstoff-Gemisch ortsselektiv im Basismaterial inkorporiert wird. Zusätzlich ist eine Verfahrbarkeit des Inkjet-Druckkopfs in einer weiteren Längsrichtung (z-Richtung) denkbar.

Das Wirkstoff-Gemisch weist beispielsweise mindestens ein Monomer und/oder mindestens ein Polymer auf. Weiterhin kann das Wirkstoff-Gemisch bevorzugt mindestens einen Photoinitiator und/oder mindestens einen Vernetzer aufweisen. Photoinitiatoren sind chemische Verbindungen, die nach Absorption von Licht in reaktive Moleküle zerfallen, die ihrerseits die Polymerisationsreaktion auslösen. Vernetzer sind Monomere oder Polymere mit mindestens drei Funktionalitäten (mehr als zwei funktionellen Gruppen oder mehr als eine Doppelbindung) die durch Polymerisationsreaktionen reagieren können. Diese bewirken eine Kettenverzweigung und damit eine Vernetzung der kettenförmigen Polymermoleküle.

In einer alternativen Ausgestaltung kann auch oder nur das Basismaterial mindestens einen Photoinitiator und/oder mindestens einen Vernetzer aufweisen. Das Basismaterial mit einem Vernetzer auszustatten bewirkt, dass das Wirkstoff-Gemisch nicht extra Vernetzer aufweisen muss. Durch die Polymerisation des Basismaterials wird der Wirkstoff mit dem Basismaterial vernetzt.

Das Monomer des Basismaterials und/oder des Wirkstoff-Gemisches ist bevorzugt ein Photomonomer. Das Polymer des Basismaterials und/oder des Wirkstoff-Gemisches ist bevorzugt ein Photopolymer. Photomonomere bzw. Photopolymere polymerisieren, wenn diese mit einer gewissen Strahlungsintensität bestrahlt werden.

Das Monomer des Basismaterials und/oder des Wirkstoffgemisches ist bevorzugt ein Vernetzer. Alternativ oder zusätzlich ist das Polymer des Basismaterials und/oder des Wirkstoffgemisches ein Vernetzer/Vernetzer ist. Das Basismaterial als Vernetzer auszubilden bewirkt, dass das Wirkstoff-Gemisch nicht extra Vernetzer aufweisen muss. Durch die Polymerisation des Basismaterials wird der Wirkstoff mit dem Basismaterial vernetzt.

Das Monomer des Basismaterials und/oder des Wirkstoff-Gemisches und/oder das Polymer des Basismaterials und/oder des Wirkstoff-Gemisches kann auch eine definierte Menge an Photoinitiatoren aufweisen.

In einer bevorzugten Ausgestaltung des erfindungsgemäßen Verfahrens, bewirkt nach dem Auftragen des Wirkstoff-Gemisches die Strahlungsquelle eine Polymerisation des Wirkstoff-Gemisches und/oder eine Vernetzung des Wirkstoff-Gemisches mit dem Basismaterial. Hierfür kann auch eine weitere Strahlungsquelle verwendet werden, wobei die erste Strahlungsquelle das Basismaterial und die weitere Strahlungsquelle das Wirkstoff-Gemisch polymerisiert. Eine Polymerisation führt bevorzugt auch zu einer Vernetzung des Basismaterials mit dem Wirkstoff.

Bevorzugt erwärmt der Inkjet-Druckkopf das Wirkstoff-Gemisch. Das Wirkstoff-Gemisch kann in einem Reservoir am Inkjet-Druckkopf angebracht sein. Es ist auch denkbar, dass das Reservoir erwärmt wird, um ein fließfähige Flüssigkeit bereitzustellen.

Weiterhin ist an dem Inkjet-Druckkopf ein Reservoir derart angebracht, dass ein Nachfließen des Wirkstoff-Gemischs erreicht wird. Hierfür kann beispielsweise das Reservoir oberhalb des Inkjet-Druckkopfs angebracht sein. Mittels dieser Anordnung wird ein Überdruck erzeugt, mit welchem ein Nachfließen sichergestellt wird. Das Reservoir kann aber auch neben oder unter dem Inkjet-Druckkopf angebracht sein, auch andere Positionen am Inkjet-Druckkopf sind denkbar.

Bevorzug ist eine Strahlungsintensität der ersten Strahlungsquelle und/oder der weiteren Strahlungsquelle einstellbar. Durch verschiedene Strahlungsintensitäten wird erreicht, dass verschiedene Wirkstoff-Gemische und/oder das Basismaterial bei voneinander verschiedenen Strahlungsintensitäten vernetzt werden können. Die Vernetzung basiert auf spaltbaren Bindungen, beispielsweise hydrolytisch spaltbaren Bindungen, wodurch sich die Spaltung der Bindungen und somit die Wirkstofffreigabe steuern lässt. Bevorzugt ist die Strahlungsintensität der ersten und/oder der weiteren Strahlungsquelle mittels einer Steuerung, welche die erste und/oder die weitere Strahlungsquelle ansteuert, einstellbar.

Bevorzugt sind die erste Strahlungsquelle und die weitere Strahlungsquelle derart ausgestaltet, dass diese voneinander verschiedene Wellenlängen erzeugen, mit welchen diese die Polymere und/oder Monomere des Basismaterials und/oder des mindestens einen Wirkstoff-Gemisches bestrahlen. Jedes Monomer oder Polymer polymerisiert bei unterschiedlichen Wellenlängen. Dass die Strahlungsquellen unterschiedliche Wellenlängen erzeugen, bewirkt, dass unterschiedliche Polymere oder Monomere für das Basismaterial als auch für das Wirkstoff-Gemisch oder die Wirkstoff-Gemische verwendet werden können. Dies wiederum bewirkt eine gesteuerte Polymerisation, was zu einer gezielten Freisetzung des Wirkstoffes führt.

Beispiele für Polymere, die in der Vorrichtung bzw. in dem Verfahren verwendet werden können, sind kommerziell erhältliche Polyethylenglycole, Poly(ethylenglycol)diacrylate und Gelatinemethacrylate. Andere Polymere sind für die Verwendung in der Vorrichtung bzw. in dem Verfahren denkbar.

Beispiele für Photoinitiatoren, welche in dem erfindungsgemäßen Verfahren oder in der Vorrichtung verwendet werden, sind 2-Hydroxy-4-(2-hydroxyethoxy)-2-methylpropiophenone oder Lithium-phenyl-2,4,6-trimethylbenzoylphosphinate (LAP).

Das Wirkstoff-Gemisch oder die Wirkstoff-Gemische, welche in der Vorrichtung oder in dem Verfahren verwendet werden, können weitere Bestandteile aufweisen, beispielsweise Lösungsmittel, Vorläuferdrogen und/oder Zusätze, welche die Stabilität des Wirkstoff-Gemisches erhöhen.

Wirkstoffe in der Vorrichtung und/oder in dem Verfahren sind bevorzugt pharmazeutische Wirkstoffe. Es können aber auch Proteine, Gifte oder Substanzen, welche für Analyseaufgaben eingesetzt werden, verwendet werden. Weitere Arten von Wirkstoffen sind denkbar.

Die erfindungsgemäße Vorrichtung sowie das erfindungsgemäße Verfahren weisen mehrere Vorteile gegenüber dem Stand der Technik auf.

Die Erfindung stellt eine Vorrichtung bereit, welche die Nachteile des Standes der Technik beseitigt und den Bau von hochauflösenden formbeliebigen Wirkstofffreisetzungssystemen mit ortsselektiven Wirkstoffdepots zulässt. Der Erfindung stellt auch ein Verfahren bereit, welches die Nachteile des Standes der Technik beseitigt und den Bau von hochauflösenden formbeliebigen Wirkstofffreisetzungssystemen mit ortsselektiven Wirkstoffdepots zulässt.

Der oder die Wirkstoffe können in den Depots unterschiedlich inkorporiert werden. Die Wirkstoffe können unvernetzt in die Basisstruktur des Wirkstofffreisetzungssystem eingebunden werden oder aber auch in unterschiedlichen Vernetzungsgraden und kovalenten Bindungen mit der Basisstruktur verbunden werden, wodurch sich eine gezielte Freisetzungskinetik erreichen lässt. Durch die Ortsselektivität und die Anzahl der Vernetzungen bzw. Bindungen werden gezielt Diffusionswege für den Wirkstoff geschaffen. Zusammen mit der individuellen Abbaukinetik wird die Freisetzung der Wirkstoffe gesteuert. Hiermit lassen sich sowohl lange Freisetzungszeiträume von 6 - 12 Monaten erreichen, aber auch längere oder deutlich kürzere Freisetzungszeiträume einstellen. Diese gezielte Wirkstofffreigabe/steuerbare Wirkstofffreisetzung wird durch verschiedene Vernetzungsgrade und/oder gezielte steuerbare Vernetzung ermöglicht.

Je nach Zusammensetzung des Photopolymers (z.B. Anteil der Photoinitiatoren) sind verschiedene Strahlungsquellen oder unterschiedliche Strahlungsintensitäten einer Quelle zur Vernetzung des Wirkstoffs mit dem Basispolymer geeignet. Die simultane oder gestaffelte Nutzung von mehreren Strahlungsquellen, als auch die Nutzung unterschiedlicher Strahlungsintensitäten einer Strahlungsquelle ist zur Steuerung der Polymerisation und der Vernetzung des Wirkstoffes mit dem Basismaterial -möglich. Mehrere Strahlungsquellen können mit derselben oder mit unterschiedlichen Wellenlängen betrieben werden. In diesem Kontext können unterschiedliche, auf die jeweiligen Wellenlängen abgestimmte Photoinitiatoren Bestandteile des Photopolymers sein. Das neu entwickelte Verfahren ermöglicht in diesem Kontext ein simultanes oder gestaffeltes Arbeiten von Inkjet-Druckköpfen und Strahlungsquellen.

Das Verfahren lässt einen hochauflösenden Bau zu. Es lassen sich Grundstrukturen erzeugen, die während des Bauprozesses mit einem Wirkstoff oder einem Wirkstoff-Polymer-Gemisch durch den Inkjet-Druckkopf beladen werden können. In dem neu entwickelten Verfahren kann der Wirkstoff oder das Wirkstoff-Gemisch gezielt in das Basismaterial inkorporiert werden. Dies erfolgt über eine gesteuerte Photopolymerisation. Es kann einer oder mehrere Wirkstoffe mit der Grundstruktur des Basispolymers gezielt vernetzt werden durch die Verwendung unterschiedlicher Vernetzer im Wirkstoff-Gemisch. Der Wirkstoff kann jedoch auch unvernetzt in die Grundstruktur des Basispolymers eingebunden werden. Somit kann die Wirkstofffreisetzung des Wirkstofffreisetzungssystems gezielt eingestellt werden.

Es können mehrere unterschiedliche Strahlungsquellen simultan oder gestaffelt arbeiten. Die Intensität einer Strahlungsquelle ist justierbar. Der Energieeintrag zur Polymerisation ist damit einstellbar. Zur Polymerisationssteuerung kann auch die Zusammensetzung der Photopolymergemische variiert werden. Beispielsweise können die Spezifikation und Menge von verwendeten Vernetzern und Photoinitiatoren verändert werden. So sind beispielsweise je nach Photoinitiator unterschiedliche Strahlungsintensitäten zur Vernetzung des Wirkstoffs mit dem Basismaterial geeignet.

Das mit dem Verfahren hergestellte Wirkstofffreisetzungssystemkann nach dem Bauprozess mit Hilfe geeigneter Waschlösungen gereinigt und ggfls. in einem Nachbereitungsschritt mittels einer Stahlungsquelle durch flächige Bestrahlung nachvernetzt werden.

Das mit dem Verfahren hergestellte Wirkstofffreisetzungssystem ist formbeliebig und die Wirkstoffdepots sind ortsbeliebig sowie in beliebiger Anzahl einbringbar. Hiermit lassen sich die Diffusionswege des Wirkstoffes gestalten. Zusammen mit der individuellen Abbaukinetik der kovalenten Bindungen und dem Vernetzungsgrad wird die Freisetzung der Wirkstoffe gesteuert. Zusätzlich charakterisiert das Verfahren ein geringer Wärmeeintrag, was eine schonende Wirkstoffverarbeitung ermöglicht.

### Ausführung der Erfindung

Die Erfindung wird anhand von Ausführungsbeispielen erläutert. Hierzu zeigen
- Figur 1: Vorrichtung nach Anspruch 1 bzw. Vorrichtung zur Durchführung eines Verfahrens nach Anspruch 17
- Figur 2: Wirkstofffreisetzungssystem hergestellt durch ein Verfahren nach Anspruch 17 bzw. hergestellt mittels einer Vorrichtung nach Anspruch 1
- Figur 3: Wirkstofffreisetzungssystem mit Vernetzer hergestellt mittels eines Verfahrens nach Anspruch 17 bzw. hergestellt mittels einer Vorrichtung nach Anspruch 1

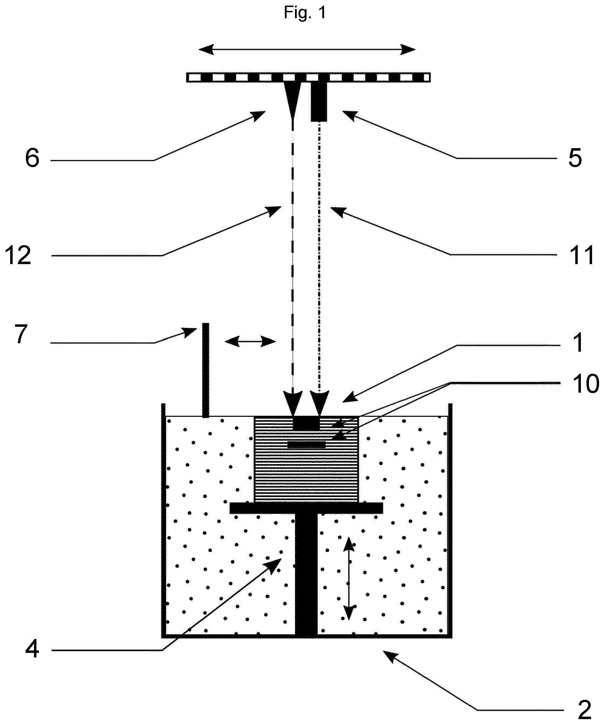

In Figur 1 ist eine Vorrichtung zur Herstellung eines 3D-gedruckten Wirkstofffreisetzungssystem 1 mit Wirkstoffdepots 10. Die Vorrichtung weist, wie in Figur 1 dargestellt, mindestens ein Inkjet-Druckkopf 6 und mindestens eine Strahlungsquelle 5 auf. Weiterhin weist die Vorrichtung eine Wanne 2 zur Aufnahme des zu verarbeitenden Basismaterials 3 auf, wobei das Basismaterial 3 ein Monomer und/oder ein Polymer ist. Eine Bauplattform 4 ist innerhalb der Wanne derart angeordnet, dass die Bauplattform 4 in vertikaler Richtung beweglich gelagert ist. Die mindestens eine Strahlungsquelle 5 ist derart ausgebildet, dass diese einen Strahl auf das Basismaterial aussendet, welcher ortsselektiv eine Polymerisation ausgewählter Bereiche einer Schicht des Basismaterials 3 auf der Bauplattform 4 bewirkt. Erfindungsgemäß ist der mindestens eine Inkjet-Druckkopf 6 derart ausgebildet, dass dieses ein Wirkstoff-Gemisch mittels eines dosierten Strahls 12 in das Basismaterial 3 ortsselektiv inkorporiert/einbringt. Das Wirkstoff-Gemisch weist mindestens einen Wirkstoff auf.

In Figur 1 ist auch eine Vorrichtung dargestellt, mit welcher das erfindungsgemäße Verfahren zur Herstellung eines 3D-gedruckten Wirkstofffreisetzungssystems mit Wirkstoffdepots 10 durchführbar ist. Das Verfahren weist folgende Verfahrensschritte auf:
Befüllen einer Wanne 2 mit einem Basismaterial 3, wobei das Basismaterial 3 ein Monomer und/oder Polymer ist,
Polymerisieren ausgewählter Bereiche einer Schicht des Basismaterials 3 mittels einer Strahlungsquelle 5 auf einer Bauplattform 4, wobei die Bauplattform 4 innerhalb der Wanne 2 derart angeordnet ist, dass die Bauplattform 4 innerhalb der Wanne 2 in vertikaler Richtung (dargestellte Pfeilrichtung) bewegbar gelagert ist,
Einbringen eines Wirkstoff-Gemisches mittels eines dosierten Strahls, welches mindestens einen Wirkstoff aufweist, mittels eines Inkjet-Druck-Verfahrens. Hierfür wird ein Inkjet-Druckkopf 6 verwendet, welcher das Wirkstoff-Gemisch ortsselektiv in das Basismaterial 3 -und/oder in die polymerisierte Schicht des Basismaterial inkorporiert.

Absenken der Bauplattform 4, wobei die Bauplattform umgebenes Basismaterial 3, welches nicht polymerisiert ist, nachfließt.

Durch das Absenken der Bauplattform können weitere ausgewählte Bereiche einer weiteren Schicht polymerisiert werden. Dadurch ist ein schichtweiser Aufbau eines dreidimensionalen Körpers, welcher die Grundstruktur des Wirkstofffreisetzungssystems bildet, möglich. Durch das Einbringen des Wirkstoff-Gemisches in ausgewählte Bereiche des dreidimensionalen Körpers werden Wirkstoffdepots 10 bereitgestellt.

Nach dem Absenken der Bauplattform, fließt das die Bauplattform umgebene Basismaterial 3 nach. Dieses kann in einem Verfahrensschritt geglättet werden.

In einer Ausgestaltung des Verfahrens bewirkt/bewirken nach dem Auftragen des Wirkstoff-Gemisches die Strahlungsquelle 5 und/oder eine zweite und/oder weitere Strahlungsquellen eine Polymerisation des Wirkstoff -Gemisches und/oder eine Vernetzung des Wirkstoff- Gemisches mit dem Basismaterial.

Zusätzlich zu den beschriebenen Verfahrensschritten erwärmt in einer weiteren Ausgestaltung der Inkjet-Druckkopf das Wirkstoff-Gemisch.

In Figur 1 ist zusätzlich ein Rakel 7 abgebildet. Diese Ausgestaltung der Vorrichtung erlaubt eine Glättung des Basismaterials 3.

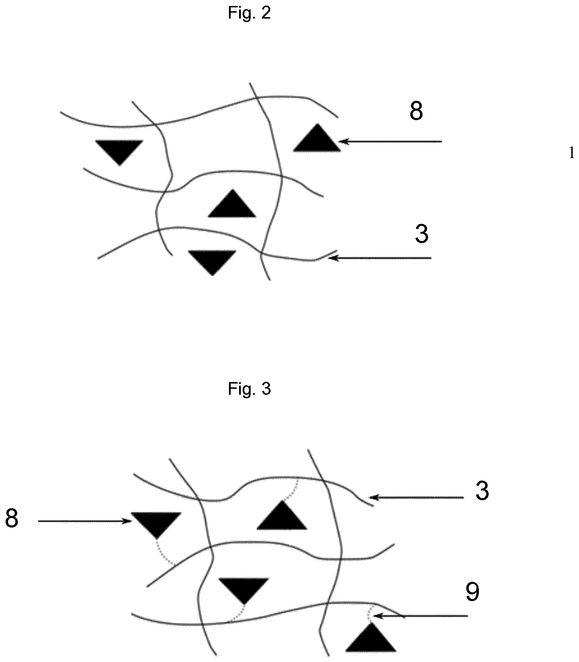

In Figur 2 ist ein Wirkstofffreisetzungssystem 1, hergestellt nach dem erfindungsgemäßen Verfahren und/oder mit einer erfindungsgemäßen Vorrichtung dargestellt. Figur 2 zeigt ein unvernetztes Wirkstofffreisetzungssystem 1. In ein Basismaterial 3 ist ein Wirkstoff 8 inkorporiert.

In Figur 3 ist ein Wirkstofffreisetzungssystem 1, hergestellt nach dem erfindungsgemäßen Verfahren und/oder mit einer erfindungsgemäßen Vorrichtung dargestellt. Figur 3 zeigt ein vernetztes Wirkstofffreisetzungssystem 1. In ein Basismaterial 3 ist ein Wirkstoff 8 inkorporiert. Der Wirkstoff 8 ist mit Basismaterial 3 vernetzt. Dies wird erreicht durch Vernetzer, welche in dem Wirkstoff-Gemisch und/oder dem Basismaterial 3 vorhanden sind. Mittels einer Strahlungsquelle 5 und einer Strahlungsintensität werden die Vernetzer aktiviert. Durch Variation der Strahlungsintensität sowie der Wahl des Vernetzers und seiner Konzentration sind unterschiedliche Vernetzungsgrade erreichbar. Diese bewirken eine gezielte Freisetzung des Wirkstoffes im Wirkstofffreisetzungssystem.

In allen Ausführunsformen kann das Wirkstoff-Gemisch und/oder das Basismaterial wie folgt ausgestaltet sein:

Das Wirkstoff-Gemisch kann ein Monomer und/oder ein Polymer aufweisen. Außerdem kann das Wirkstoff-Gemisch einen Photoinitiator und/oder einen Vernetzer aufweisen. Dies erlaubt eine Polymerisation des Wirkstoff-Gemisches, was wiederum zu einer erhöhten Stabilität des Wirkstoff-Gemisches, einer Vernetzung des Wirkstoff-Gemisches mit dem Basismaterial, sowie einer variierenden Diffusion des Wirkstoffes erlaubt.

Auch das Basismaterial 3 oder nur das Basismaterial 3 kann mindestens einen Photoinitiator und/oder mindestens einen Vernetzer aufweisen.

Das Monomer des Basismaterials und/oder des Wirkstoff-Gemisches kann als ein Photomonomer ausgestaltet sein und/oder das Polymer des Basismaterials und/oder des Wirkstoff-Gemisches kann als ein Photopolymer ausgestaltet sein.

Das Monomer des Basismaterials und/oder des Wirkstoffgemisches und/oder das Polymer des Basismaterials und/oder des Wirkstoffgemisches kann/können als Vernetzer ausgestaltet sein.

Das Monomer des Basismaterials und/oder des Wirkstoff-Gemisches und/oder das Polymer des Basismaterials und/oder des Wirkstoff-Gemisches kann zudem eine definierte Menge an Photoinitiatoren aufweisen.

In allen Ausführungsformen kann die Strahlungsquelle 5 und/oder eine weiter Strahlungsquelle wie folgt ausgestaltet sein:

Die erste Strahlungsquelle 5 ist in Figur 1 derart ausgebildet, dass diese in Betrieb eine Polymerisation des Wirkstoff-Gemisches bewirkt. Alternativ oder zusätzlich bewirkt die Strahlungsquelle 5 eine Vernetzung des Wirkstoff-Gemisches mit dem Basismaterial 3.

In einer weiteren Ausgestaltung ist eine zweite Strahlungsquelle angeordnet (hier nicht abgebildet). Diese bewirkt bei Benutzung eine Polymerisation des Wirkstoff- Gemisches und/oder eine Vernetzung des Wirkstoff-Gemisches mit dem Basismaterial 3.

Die Strahlungsquelle 5 ist als ein Laser, eine Laserdiode, eine Lichtdiode oder eine Maskenbelichtung ausgebildet. Die zweite Strahlungsquelle kann ebenfalls als ein Laser, eine Laserdiode, eine Lichtdiode oder eine Maskenbelichtung ausgebildet sein. Um unterschiedliche Strahlungsintensitäten und -wellenlängen zwischen verschiedenen Strahlungsqullen zu erreichen kann die zweite Strahlungsquellen eine zur ersten Strahlungsquelle verschiedene Ausführung sein. Beispielsweise ist die Strahlungsquelle 5 eine Laserdiode und die zweite Strahlungsquelle eine Maskenbelichtung. Andere Kombinationen sind denkbar.

Die Strahlungsquelle 5 und/oder die zweite Strahlungsquelle sind in einer Ausführungsform derart ausgestaltet, dass diese in ihrer Strahlungsintensität einstellbar ist/sind.

Die Strahlungsquelle 5 und die zweite Strahlungsquelle sind in einer Ausführungsform derart ausgestaltet, dass diese voneinander verschiedene Wellenlängen erzeugen.

In allen Ausführungsformen kann der Inkjet-Druckkopf 6 wie folgt ausgestaltet sein:

Der Inkjet-Druckkopf 6 ist in einer Ausführungsform beheizbar. Hierfür weist dieser eine Heizvorrichtung auf.

Der Inkjet-Druckkopf 6 weist in einer Ausführungsform ein Reservoir (hier nicht abgebildet) zur Aufnahme des Wirkstoff-Gemisches auf. Das Reservoir kann beheizbar ausgestaltet sein. Hierfür weist das Reservoir eine Heizvorrichtung auf.

Um ein Nachfließen des Wirkstoff-Gemisches zu gewährleisten, kann das Reservoir derart an dem Inkjet-Druckkopf 6 angebracht seinist, dass ohne zusätzliche Kräfte, bespielsweise durch Pumpen erzeugte Kräfte, nur mittels Naturkräfte ein Nachfließen des Wirkstoff-Gemisches erreicht wird. Naturkräfte können beispielsweise Gravitationskräfte oder Kapillarkräfte sein.

Da es sich bei der vorhergehenden, detailliert beschriebenen Vorrichtung und dem Verfahren um Ausführungsbeispiele handelt, können sie in üblicher Weise vom Fachmann in einem weiten Umfang modifiziert werden, ohne den Bereich der Erfindung zu verlassen. Insbesondere können auch die konkreten Ausgestaltungen der Vorrichtung in anderer Form als in der hier beschriebenen erfolgen. Ebenso kann die Vorrichtung in einer anderen Form ausgestaltet werden, wenn dies aus Platzgründen bzw. designerischen Gründen notwendig ist. Weiter schließt die Verwendung der unbestimmten Artikel "ein" bzw. "eine" nicht aus, dass die betreffenden Merkmale auch mehrfach vorhanden sein können.

### Bezugszeichenliste

- 1: Wirkstofffreisetzungssystem
- 2: Wanne
- 3: Basismaterial
- 4: Bauplattform
- 5: Strahlungsquelle
- 6: Inkjet-Druckkopf
- 7: Rakel
- 8: Wirkstoff
- 9: Vernetzer
- 10: Wirkstoffdepot
- 11: Strahl
- 12: Dosierter Strahl des Wirkstoff-Gemisches

## Patentansprüche

1. Vorrichtung zur Herstellung eines 3D-gedruckten Wirkstofffreisetzungssystem (1) mit Wirkstoffdepots (10), aufweisend mindestens einen Inkjet-Druckkopf (6) und eine erste Strahlungsquelle (5), eine Wanne (2), in welcher ein zu verarbeitendes Basismaterials (3) angeordnet ist, eine Bauplattform (4),
wobei die Bauplattform (4) innerhalb der Wanne (2) beweglich gelagert ist, wobei die Vorrichtung derart ausgebildet ist, dass die erste Strahlungsquelle -(5) in Betrieb eine Polymerisation ausgewählter Bereiche des Basismaterials (3) auf der Bauplattform (4) bewirkt,
wobei
die Vorrichtung derart ausgebildet ist, dass der mindestens eine Inkjet-Druckkopf (6) in Betrieb ein Wirkstoff-Gemisch in das Basismaterial (3) auf der Bauplattform (3) ortsselektiv inkorporiert, wobei das Wirkstoff-Gemisch mindestens einen Wirkstoff (8) aufweist.

2. Vorrichtung nach Anspruch 1 wobei
das Wirkstoff-Gemisch mindestens ein Monomer und/oder mindestens ein Polymer und/oder mindestens einen Photoinitiator und/oder mindestens einen Vernetzer (9) aufweist.

3. Vorrichtung nach Anspruch 1 wobei
das Basismaterial (3) mindestens einen Photoinitiator und/oder mindestens einen Vernetzer (9) aufweist.

4. Vorrichtung nach Anspruch 1 oder 2 wobei
das Monomer des Basismaterials und/oder des Wirkstoff-Gemisches ein Photomonomer ist und/oder das Polymer des Basismaterials und/oder des Wirkstoff-Gemisches ein Photopolymer ist.

5. Vorrichtung nach einem der vorhergehenden Ansprüche, wobei
das Monomer des Basismaterials und/oder des Wirkstoff-Gemisches und/oder 07.09.2020
das Polymer des Basismaterials und/oder des Wirkstoff-Gemisches ein Vernetzer/Vernetzer (9) ist/sind und/oder eine definierte Menge an Photoinitiatoren aufweist/aufweisen.

6. Vorrichtung nach einem der Ansprüche 1-5 wobei
die Vorrichtung derart ausgebildet ist, dass die erste Strahlungsquelle (5) eine Polymerisation des Wirkstoff-Gemisches bewirkt und/oder eine Vernetzung des Wirkstoff-Gemisches mit dem Basismaterial (3) bewirkt.

7. Vorrichtung nach einem der Ansprüche 1-6, wobei
eine weitere Strahlungsquelle angeordnet ist und dass die Vorrichtung derart ausgebildet ist, dass die weitere Strahlungsquelle in Betrieb eine Polymerisation des Wirkstoff- Gemisches bewirkt und/oder eine Vernetzung des Wirkstoff-Gemisches mit dem Basismaterial (3) bewirkt.

8. Vorrichtung nach einem der vorhergehenden wobei
der erste Inkjet-Druckkopf (6) eine Heizvorrichtung aufweist.

9. Vorrichtung nach einem der vorhergehenden Ansprüche, wobei
der mindestens eine Inkjet-Druckkopf (6) ein Reservoir zur Aufnahme des Wirkstoff-Gemisch aufweist.

10. Vorrichtung nach Anspruch 9 wobei
das Reservoir eine Heizvorrichtung aufweist.

11. Vorrichtung nach Anspruch 9 oder 10 wobei
das Reservoir derart an dem mindestens einen Inkjet-Druckkopf (6) angebracht ist, dass ein Nachfließen des Wirkstoff-Gemischs erreicht wird.

12. Vorrichtung nach einem der vorhergehenden Ansprüche wobei die erste Strahlungsquelle (5) und/oder die weitere Strahlungsquelle ein optoelektrisches Bauelement und/oder ein optischen Resonator aufweist.

13. Vorrichtung nach einem der vorhergehenden Ansprüche wobei
die erste Strahlungsquelle (5) und/oder die weitere Strahlungsquelle verschiedene Strahlungscharakteristiken aufweisen.

14. Vorrichtung nach einem der vorhergehenden Ansprüche wobei
die Strahlungsquelle (5) und die zweite Strahlungsquelle derart ausgestaltet ist/sind, dass diese voneinander verschiedene Wellenlängen erzeugen.

15. Vorrichtung nach einem der vorhergehenden Ansprüche, ferner aufweisend zumindest einen weiteren Inkjet-Druckkopf, welcher derart ausgebildet ein Wirkstoff-Gemisch ortsselektiv in das Basismaterial zu inkorporieren.

16. Vorrichtung nach Anspruch 15, wobei
das Wirkstoff-Gemisch des zumindest einen weiteren Inkjet-Druckkopfes verschieden ist zu dem Wirkstoff-Gemisch des ersten Inkjet-Druckkopfes.

17. Verfahren zur Herstellung eines 3D-gedruckten Wirkstofffreisetzungssystems (1), aufweisend folgende Verfahrensschritte:
• Bereitstellen einer Wanne (2), in welcher ein Basismaterial (3) und eine Bauplattform angeordnet sind, wobei das Basismaterial (3) ein Monomer und/oder Polymer ist,
• Polymerisieren ausgewählter Bereiche einer Schicht des Basismaterials (3) auf der Bauplattform (4),
wobei
• mindestens ein Wirkstoff-Gemisch, welches mindestens einen Wirkstoff (8) aufweist, mittels eines Inkjet-Druck-Verfahrens (6) ortsselektiv in das Basismaterial (3) und/oder das polymerisierte Basismaterial (3) inkorporiert wird.

18. Verfahren nach Anspruch 17 wobei
das Wirkstoff-Gemisch mindestens ein Monomer und/oder mindestens ein Polymer und/oder mindestens einen Photoinitiator und/oder mindestens einen Vernetzer (9) aufweist.

19. Verfahren nach Anspruch 17, wobei
das Basismaterial (3) mindestens einen Photoinitiator und/oder mindestens einen Vernetzer (9) aufweist.

20. Verfahren nach Anspruch 17, 18 oder 19, wobei
das Monomer des Basismaterials und/oder des Wirkstoff-Gemisches ein Photomonomer ist und/oder das Polymer des Basismaterials und/oder des Wirkstoff-Gemisches ein Photopolymer ist.

21. Verfahren nach einem der Ansprüche 17 bis 20,
wobei das Monomer des Basismaterials und/oder des Wirkstoffgemisches und/oder das Polymer des Basismaterials und/oder des Wirkstoffgemisches ein Vernetzer/Vernetzer (9) ist/sind und/oder
eine definierte Menge an Photoinitiatoren aufweist/aufweisen.

22. Verfahren nach einem der Ansprüche 17 bis 21, wobei
nach dem Inkorporieren des Wirkstoff-Gemisches eine Polymerisation des Wirkstoff -Gemisches und/oder eine Vernetzung des Wirkstoff- Gemisches mit dem Basismaterial (3) bewirkt wird.

23. Verfahren nach einem der vorhergehenden Ansprüche 17 bis 22, wobei
das das Wirkstoff-Gemisch erwärmt wird.

24. Verfahren nach einem der vorhergehenden Ansprüche 17 bis 23, wobei
ein Reservoir derart an einem Inkjet-Druckkopf (6) angebracht ist, dass ein Nachfließen des Wirkstoff-Gemischs erreicht wird.

25. Verfahren nach einem der vorhergehenden Ansprüche 17 bis 24, wobei
eine Strahlungsintensität einer ersten Strahlungsquelle (5) und/oder einer weiteren Strahlungsquelle eingestellt wird und/oder wobei die erste Strahlungsquelle (5) und die weitere Strahlungsquelle voneinander verschiedene Wellenlängen erzeugen.

## Claims

1. Device for producing a 3D-printed active substance releasing system (1) with active substance depots (10), having at least one inkjet print head (6) and a first radiation source (5), a trough (2), in which a base material (3) to be processed is arranged, a construction platform (4), wherein the construction platform (4) is movably mounted within the trough (2), wherein the device is formed in such a way that the first radiation source (5) effects polymerization of selected regions of the base material (3) on the construction platform (4) during operation, wherein the device is formed in such a way that the at least one inkjet print head (6) incorporates an active substance mixture in the base material (3) on the construction platform (3) in a locally selective manner during operation, wherein the active substance mixture has at least one active substance (8).

2. Device according to Claim 1, wherein the active substance mixture has at least one monomer and/or at least one polymer and/or at least one photoinitiator and/or at least one cross-linker (9).

3. Device according to Claim 1, wherein the base material (3) has at least one photoinitiator and/or at least one cross-linker (9).

4. Device according to Claim 1 or 2, wherein the monomer of the base material and/or of the active substance mixture is a photomonomer and/or the polymer of the base material and/or of the active substance mixture is a photopolymer.

5. Device according to one of the preceding claims, wherein the monomer of the base material and/or of the active substance mixture and/or the polymer of the base material and/or of the active substance mixture is/are a cross-linker/cross-linkers (9) and/or has/have a defined quantity of photoinitiators.

6. Device according to one of Claims 1-5, wherein the device is formed in such a way that the first radiation source (5) effects polymerization of the active substance mixture and/or cross-linking of the active substance mixture with the base material (3).

7. Device according to one of Claims 1-6, wherein a further radiation source is arranged, and in that the device is formed in such a way that the further radiation source effects polymerization of the active substance mixture and/or cross-linking of the active substance mixture with the base material (3) during operation.

8. Device according to one of the preceding claims, wherein the first inkjet print head (6) has a heating device.

9. Device according to one of the preceding claims, wherein the at least one inkjet print head (6) has a reservoir to hold the active substance mixture.

10. Device according to Claim 9, wherein the reservoir has a heating device.

11. Device according to Claim 9 or 10, wherein the reservoir is attached to the at least one inkjet print head (6) in such a way that the active substance mixture continues to flow.

12. Device according to one of the preceding claims, wherein the first radiation source (5) and/or the further radiation source has an optoelectronic component and/or an optical resonator.

13. Device according to one of the preceding claims, wherein the first radiation source (5) and/or the further radiation source have different radiation characteristics.

14. Device according to one of the preceding claims, wherein the radiation source (5) and the second radiation source is/are configured in such a way that these produce mutually different wavelengths.

15. Device according to one of the preceding claims, further having at least one further inkjet print head, which is formed in such a way as to incorporate an active substance mixture in the base material in a locally selective manner.

16. Device according to Claim 15, wherein the active substance mixture from the at least one further inkjet print head is different from the active substance mixture from the first inkjet print head.

17. Method for producing a 3D-printed active substance releasing system (1), having the following method steps:
• providing a trough (2) in which a base material (3) and a construction platform are arranged, wherein the base material (3) is a monomer and/or polymer,
• polymerizing selected regions of a layer of the base material (3) on the construction platform (4),
wherein
• at least one active substance mixture which has at least one active substance (8) is incorporated in the base material (3) and/or the polymerized base material (3) in a locally selective manner by means of an inkjet printing method (6).

18. Method according to Claim 17, wherein the active substance mixture has at least one monomer and/or at least one polymer and/or at least one photoinitiator and/or at least one cross-linker (9).

19. Method according to Claim 17, wherein the base material (3) has at least one photoinitiator and/or at least one cross-linker (9).

20. Method according to Claim 17, 18 or 19, wherein the monomer of the base material and/or of the active substance mixture is a photomonomer and/or the polymer of the base material and/or of the active substance mixture is a photopolymer.

21. Method according to one of Claims 17 to 20, wherein the monomer of the base material and/or of the active substance mixture and/or the polymer of the base material and/or of the active substance mixture is/are a cross-linker/cross-linkers (9) and/or has/have a defined quantity of photoinitiators.

22. Method according to one of Claims 17 to 21, wherein following the incorporation of the active substance mixture, polymerization of the active substance mixture and/or cross-linking of the active substance mixture with the base material (3) is effected.

23. Method according to one of the preceding Claims 17 to 22, wherein the active substance mixture is heated.

24. Method according to one of the preceding Claims 17 to 23, wherein a reservoir is attached to an inkjet print head (6) in such a way that the active substance mixture continues to flow.

25. Method according to one of the preceding Claims 17 to 24, wherein a radiation intensity of a first radiation source (5) and/or of a further radiation source is set and/or wherein the first radiation source (5) and the further radiation source produce mutually different wavelengths.

## Revendications

1. Dispositif pour la fabrication d'un système de libération de substance active imprimé en 3D (1) comprenant des dépôts de substance active (10), présentant au moins une tête d'impression à jet d'encre (6) et une première source de rayonnement (5), une cuve (2), dans laquelle est agencé un matériau de base à traiter (3), une plate-forme de construction (4),
dans lequel la plate-forme de construction (4) est montée de manière mobile à l'intérieur de la cuve (2), le dispositif étant configuré de telle sorte que la première source de rayonnement (5), en fonctionnement, provoque une polymérisation de zones sélectionnées du matériau de base (3) sur la plate-forme de construction (4),
le dispositif étant configuré de telle sorte que l'au moins une tête d'impression à jet d'encre (6), en fonctionnement, incorpore de manière localisée un mélange de substance active dans le matériau de base (3) sur la plate-forme de construction (3), dans lequel le mélange de substance active présente au moins une substance active (8).

2. Dispositif selon la revendication 1, dans lequel le mélange de substance active présente au moins un monomère et/ou au moins un polymère et/ou au moins un photoinitiateur et/ou au moins un agent de réticulation (9) .

3. Dispositif selon la revendication 1, dans lequel le matériau de base (3) présente au moins un photoinitiateur et/ou au moins un agent de réticulation (9) .

4. Dispositif selon la revendication 1 ou 2, dans lequel le monomère du matériau de base et/ou du mélange de substance active est un photomonomère et/ou le polymère du matériau de base et/ou du mélange de substance active est un photopolymère.

5. Dispositif selon l'une quelconque des revendications précédentes, dans lequel le monomère du matériau de base et/ou du mélange de substance active et/ou le polymère du matériau de base et/ou du mélange de substance active est/sont un agent de réticulation/des agents de réticulation (9) et/ou présente/présentent une quantité définie de photoinitiateurs.

6. Dispositif selon l'une quelconque des revendications 1 à 5, le dispositif étant configuré de telle sorte que la première source de rayonnement (5) provoque une polymérisation du mélange de substance active et/ou provoque une réticulation du mélange de substance active avec le matériau de base (3).

7. Dispositif selon l'une quelconque des revendications 1 à 6, dans lequel une autre source de rayonnement est agencée et en ce que le dispositif est configuré de telle sorte que l'autre source de rayonnement, en fonctionnement, provoque une polymérisation du mélange de substance active et/ou provoque une réticulation du mélange de substance active avec le matériau de base (3).

8. Dispositif selon l'une quelconque des revendications précédentes, dans lequel la première tête d'impression à jet d'encre (6) présente un dispositif de chauffage.

9. Dispositif selon l'une quelconque des revendications précédentes, dans lequel l'au moins une tête d'impression à jet d'encre (6) présente un réservoir pour recevoir le mélange de substance active.

10. Dispositif selon la revendication 9, dans lequel le réservoir présente un dispositif de chauffage.

11. Dispositif selon la revendication 9 ou 10, dans lequel le réservoir est disposé sur l'au moins une tête d'impression à jet d'encre (6) de manière à obtenir un écoulement du mélange de substance active.

12. Dispositif selon l'une quelconque des revendications précédentes, dans lequel la première source de rayonnement (5) et/ou l'autre source de rayonnement présente un composant optoélectrique et/ou un résonateur optique.

13. Dispositif selon l'une quelconque des revendications précédentes, dans lequel la première source de rayonnement (5) et/ou l'autre source de rayonnement présentent des caractéristiques de rayonnement différentes.

14. Dispositif selon l'une quelconque des revendications précédentes, dans lequel la source de rayonnement (5) et la deuxième source de rayonnement sont conçues de telle sorte que celles-ci génèrent des longueurs d'onde différentes l'une de l'autre.

15. Dispositif selon l'une quelconque des revendications précédentes, présentant en outre au moins une autre tête d'impression à jet d'encre, qui est configurée pour incorporer un mélange de substance active de manière localisée dans le matériau de base.

16. Dispositif selon la revendication 15, dans lequel le mélange de substance active de l'au moins une autre tête d'impression à jet d'encre est différent du mélange de substance active de la première tête d'impression à jet d'encre.

17. Procédé de fabrication d'un système de libération de substance active imprimé en 3D (1), présentant les étapes de procédé suivantes :
- la fourniture d'une cuve (2), dans laquelle sont agencés un matériau de base (3) et une plate-forme de construction, le matériau de base (3) étant un monomère et/ou un polymère,
- la polymérisation de zones sélectionnées d'une couche du matériau de base (3) sur la plate-forme de construction (4),
dans lequel
- au moins un mélange de substance active, qui présente au moins une substance active (8), est incorporé de manière localisée dans le matériau de base (3) et/ou le matériau de base polymérisé (3) au moyen d'un procédé d'impression à jet d'encre (6).

18. Procédé selon la revendication 17, dans lequel le mélange de substance active présente au moins un monomère et/ou au moins un polymère et/ou au moins un photoinitiateur et/ou au moins un agent de réticulation (9) .

19. Procédé selon la revendication 17, dans lequel le matériau de base (3) présente au moins un photoinitiateur et/ou au moins un agent de réticulation (9) .

20. Procédé selon les revendications 17, 18 ou 19, dans lequel le monomère du matériau de base et/ou du mélange de substance active est un photomonomère et/ou le polymère du matériau de base et/ou du mélange de substance active est un photopolymère.

21. Procédé selon l'une quelconque des revendications 17 à 20, dans lequel le monomère du matériau de base et/ou du mélange de substance active et/ou le polymère du matériau de base et/ou du mélange de substance active est/sont un agent de réticulation/des agents de réticulation (9) et/ou présente/présentent une quantité définie de photoinitiateurs.

22. Procédé selon l'une quelconque des revendications 17 à 21, dans lequel, après l'incorporation du mélange de substance active, une polymérisation du mélange de substance active et/ou une réticulation du mélange de substance active avec le matériau de base (3) est provoquée.

23. Procédé selon l'une quelconque des revendications 17 à 22 précédentes, dans lequel le mélange de substance active est chauffé.

24. Procédé selon l'une quelconque des revendications 17 à 23 précédentes, dans lequel un réservoir est disposé sur une tête d'impression à jet d'encre (6) de manière à obtenir un écoulement du mélange de substance active.

25. Procédé selon l'une quelconque des revendications 17 à 24 précédentes, dans lequel une intensité de rayonnement d'une première source de rayonnement (5) et/ou d'une autre source de rayonnement est ajustée et/ou dans lequel la première source de rayonnement (5) et l'autre source de rayonnement génèrent des longueurs d'onde différentes l'une de l'autre.
